Europäisches Patentamt

(19) European Patent Office

Office européen des brevets

(11) Publication number: **0 015 897**
**B1**

(12) **EUROPEAN PATENT SPECIFICATION**

(45) Date of publication of patent specification: **20.07.83**

(21) Application number: **78900186.4**

(22) Date of filing: **19.06.78**

(86) International application number:
**PCT/SE78/00002**

(87) International publication number:
**WO 80/00067 24.01.80 Gazette 80/2**

(51) Int. Cl.³: **B 29 C 27/02, F 16 L 47/02**
**//(A61M1/03)**

(54) **METHOD OF JOINING CONDUITS WITH A CONNECTING PIECE AND CONNECTING PIECE FOR CARRYING OUT THE METHOD.**

(43) Date of publication of application:
**01.10.80 Bulletin 80/20**

(45) Publication of the grant of the patent:
**20.07.83 Bulletin 83/29**

(84) Designated Contracting States:
**CH DE FR GB LU SE**

(56) References cited:
**DE - A - 2 628 203**
**DE - B - 1 173 232**
**DE - B - 1 238 652**
**GB - A - 857 859**
**SE - B - 316 289**

(73) Proprietor: **Gambro Lundia AB**
**Box 10101**
**S-220 10 Lund (SE)**

(72) Inventor: **STENBERG, Kaj, Ove**
**Fugavägen 53**
**S-245 00 Staffanstorp (SE)**

(74) Representative: **Boberg, Nils Gunnar Erik**
**Gambro AB Patent Department Box 10101**
**S-220 10 Lund (SE)**

Courier Press, Leamington Spa, England

Method of joining conduits with a connecting piece
and connecting piece for carrying out the method

Technical Field

The invention relates to and provides a method of joining two or more conduits by means of a one-piece tubular connecting piece comprising conduit end-receiving openings in a number corresponding to the number of conduits to be joined, wherein the conduit ends are located at the respective conduit end-receiving openings, wherein support means is used to support the so located conduit ends whilst being secured to the connecting piece, and wherein the so located conduit ends are secured to the respective conduit end-receiving openings whilst being supported by the support means. The invention also provides a tubular connecting piece for carrying out the method. The invention has particular, but not exclusive, application to welding thermoplastics tubes to a thermoplastics connecting piece.

Background Art

It is well known to join two or more conduits with a tubular connecting piece by locating the conduit ends to be connected at respective conduit end-receiving openings in the connecting piece and securing said ends to the connecting piece to communicate with the respective conduit end-receiving openings. In particular, this method is used to connect thermoplastics tubes by welding the tubes to a thermoplastics connecting piece. The conduit ends require supporting whilst being secured to the connecting piece and it is known to provide this support by inserting support means into the connecting piece through one of the conduit end-receiving openings. Obviously, however, it has not been possible to support all of the conduits from inside the connecting piece because the support means must be withdrawn from said opening to allow the respective conduit end to be connected to the connecting piece to communicate with said conduit end-receiving opening. It is an object of the present invention to provide a method whereby all of the conduit ends can be supported from inside the connecting piece whilst they are secured to the connecting piece.

Disclosure of Invention

According to one aspect of the present invention, there is provided a method of joining two or more conduits by means of a one-piece tubular connecting piece comprising conduit end-receiving openings in a number corresponding to the number of conduits to be joined, wherein the conduit ends are located at the respective conduit end-receiving openings, wherein support means is used to support the so located conduit ends whilst being secured to the connecting piece, and wherein the so located conduit ends are secured to the respective conduit end-receiving openings whilst being supported by the support means. The method is characterized in that said support means is inserted into and withdrawn from, respectively, the connecting piece through a separate sealable opening in the connecting piece, which separate sealable opening is designed especially and exclusively for inserting and withdrawing, respectively, of the support, means therethrough and which separate sealable opening is then sealed, whereby all of the so located conduit ends can be secured to the respective conduit end-receiving openings whilst being supported by the support means from inside the connecting piece.

The conduit ends can be secured to the connecting piece by any of the means conventionally used for connecting conduits to a connecting piece but it is preferred that they are welded to the connecting piece. As mentioned previously, the invention has particular application to the joining of thermoplastics tubes with a thermoplastics connecting piece. Suitable thermoplastics include polyvinylchloride and polyurethane.

The support means usually, but not necessarily, will be used to support all of the conduit ends during the time that they are being secured to the connecting piece. It will be appreciated however that the conventional method of inserting support means through a conduit end-receiving opening can be employed for all except the last conduit end and the method of the invention used for securing that last conduit end to the connecting piece.

According to a second aspect of the present invention, there is provided a one-piece tubular connecting piece for use in the method of the invention, which comprises conduit end-receiving openings in a number corresponding to the number of conduits to be joined. The connecting piece is characterized by comprising a separate sealable opening which is designed especially and exclusively for inserting and withdrawing, respectively, of the support means therethrough and which is then intended to be sealed, whereby all of the so located conduit ends can be secured to the respective conduit end-receiving openings whilst being supported by the support means from inside the connecting piece.

Advantageously, the sealable opening is a slot and preferably the connecting piece is elongate and the slot extends longitudinally of the elongate connecting piece.

Flaps preferably are provided integrally with the connecting piece for use in sealing the sealable opening. When the said opening is a slot, two flaps suitably are provided to extend longitudinally at the respective sides of the slot. These two flaps preferably have plane-convex opposed (i.e. facing) surfaces whereby the said surfaces smoothly mutually diverge outwardly from the slot. It is also preferred that any flaps integral with the connecting piece are formed of thermoplastics so that the opening can be sealed by heating and pressing the flaps together over the opening.

Usually, the conduit end-receiving openings in the connecting piece will be tubular end portions of the connecting piece and the conduit ends will be inserted into or placed over the respective tubular end portions of the connecting piece.

The sealable opening ends can be located at any convenient position in the connecting piece but suitably is located opposite a conduit end-receiving to facilitate insertion of the support means into that opening.

The connecting piece can be of any suitable shape provided that it serves to connect the conduits together in the required manner. Advantageously however, the central axis of tubular end portions are substantially coplanar and the connecting piece has a "Y" or "T"-shaped axial section in said plane.

Conveniently, the support means constitutes an electrode for use in welding the respective conduit end to the connecting piece.

Brief Description of Drawings

The following is a description, by way of example only and with reference to the accompanying drawings of the presently most preferred embodiment of the method and apparatus aspects of the invention. In the drawings:

Fig. 1 is a side view of a connecting piece according to the most preferred embodiment of the invention having three tubes welded to tubular ends of the connecting piece;

Fig. 2 is an enlarged axial section of the connecting piece of Fig. 1, but without the tubes welded thereto;

Fig. 3 is a further enlarged cross-section of the connecting piece along the line III—III in Fig. 2; and

Fig. 4 is a corresponding axial section to Fig. 2 of the connecting piece of Fig. 1, but with the respective tubes being arranged in the tubular ends of the connecting piece while being supported by supporting means.

Best Mode of Carrying Out the Invention

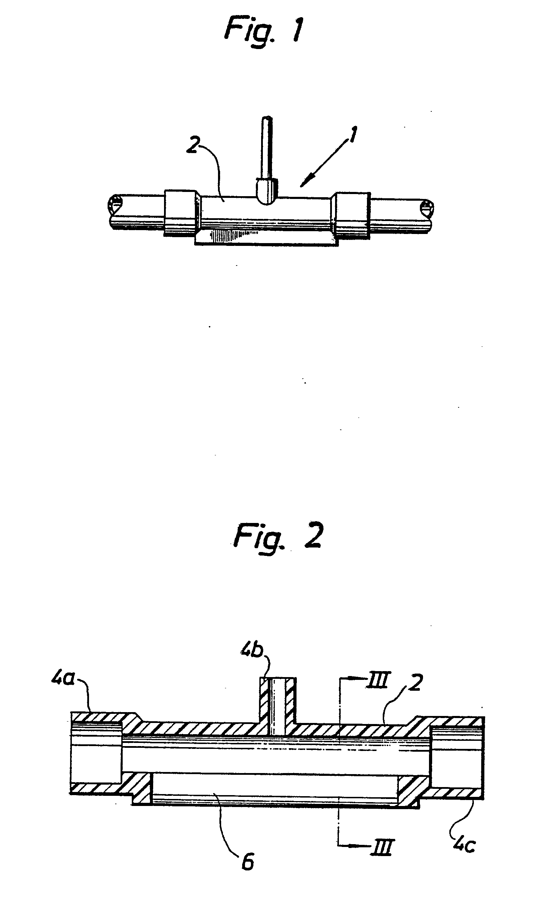

Referring to Figs. 1 to 4, a connecting piece, designated 1, comprises a tubular portion 2 having three open tubular ends 4a, 4b, 4c. The tubular portion 2 is provided with a hold 6, through which three support members 5a, 5b, 5c are intended to be inserted into the connecting piece 1 so as to support tubes 3a, 3b, 3c when these are being welded to the connecting piece.

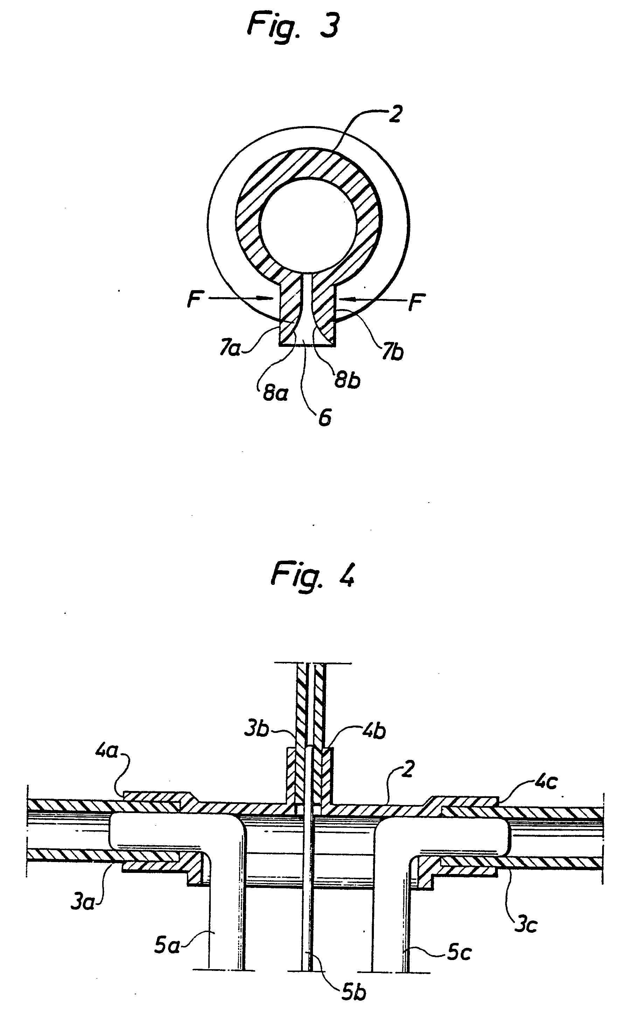

The hole 6, which is sealable, is a longitudinal slot and is provided opposite to the open tubular end 4b. The hole 6 has two parallel, longitudinal flaps 7a, 7b extending along its respective edges as shown in Fig. 3. These flaps are each integrally formed with the tubular portion 2 and have plane-convex surfaces 8a and 8b, respectively, facing each other, whereby said surfaces smoothly mutually diverge radially outwardly from the slot 6. The outer surfaces of the respective flaps 7a, 7b are planar.

The axes of the open tubular ends 4a, 4b, 4c are provided in the same plane and so that the tubular portion 2 has a T-shaped axial section in said plane.

The connecting piece 1 as well as the respective tubes are made of a weldable thermoplastic material, for example polyurethane and preferably polyvinylchloride (PVC).

As shown in Fig. 3 the tubular portion 2 is somewhat thicker just opposite to the hole 6 in order to increase the strength of the portion 2.

In welding together the respective end portions of the tubes 3a, 3b, 3c to the corresponding open tubular ends 4a, 4b, 4c of the connecting piece 1 in accordance with the present invention, separate supporting members 5a, 5b, 5c are inserted into the tubular portion 2 through the sealable hole 6 to be received in the respective tubes. It is to be noted that these supporting members (which together constitute support means) can be inserted into the tubular portion 2 at the same time, whereby all the tubes can be supported simultaneously. The supporting members 5a, 5b, 5c are so dimensioned that, in place within the tubular portion, as is shown in Fig. 4, they are closely surrounded by the tubes 3a, 3b, 3c. In that way there is provided sufficient support to the tubes.

In high frequency welding, which is the most preferred welding method according to the present invention, these supporting means may be the welding electrodes. The other electrodes (not shown), are constituted by conventional brass jaws, which externally surround the respective open tubular ends 4a, 4b, 4c of the connecting piece 1.

When the tubes as well as the welding electrodes, inclusive of the separate supporting members 5a, 5b, 5c, are in place, all the tubes can be welded simultaneously to the respective open tubular ends 4a, 4b, 4c of the connecting piece 1.

When the tubes have been welded to the tubular ends 4a, 4b, 4c of the connecting piece, the supporting members 5a, 5b, 5c are withdrawn, and the hole 6 is then sealed. The sealing is preferably performed by welding, wherein jaws (not shown) or the like, acting as electrodes, press the longitudinal flaps 7a, 7b against each other, as is shown by arrows F in Fig. 3. The flaps 7a, 7b are melted together due to the heat generated during the welding. Molten material flows radially outwardly (downwardly in Fig. 3) along the plane-convex surfaces 8a, 8b of the flaps and not into (upwardly in Fig. 3) the tubular portion 2 through the hole 6. This is due to the fact that the surfaces 8a, 8b have the plane-convex shape shown in Fig. 3. As a result there is obtained a connecting piece which, since the hole has been sealed, has smooth inner surfaces without any sharp edges.

Industrial Applicability

A preferred application of the invention will be the connecting of a heparine tube to a blood tube via a connecting piece in medical devices for, for example, extracorporeal treating of blood. In this case it is very important that the connecting piece with the tubes in place does not display any irregularities in the flow path of the blood. Sharp edges or substantially altered flowing conditions, which may lead to substantial turbulence, can be deleterious to the blood. According to the present invention there can be obtained a connecting piece without any irregularities or the like which may lead to deleterious turbulence in the blood stream.

It will be appreciated that the invention is not restricted to the details described above, for example, instead of the tubes 3a, 3b, 3c being inserted into the tubular ends 4a, 4b, 4c of the connecting piece 1 they can be welded to the connecting piece outside the end portions 4a, 4b, 4c thereof. In such a case the tubes 3a, 3b, 3c are slipped over the connecting piece 1 at the tubular end 4a, 4b, 4c portions thereof and are welded to said portions outside the connecting piece. Further, the connecting piece can be of a different shape, for example the tubular portion 2 can have a "Y"-shape in the plane of the axes of the tubular ends 4a, 4b, 4c instead of the "T"-shape shown.

**Claims**

1. A method of joining two or more conduits (3a, 3b, 3c) by means of a one-piece tubular connecting piece (1) comprising conduit end-receiving openings (4a, 4b, 4c) in a number corresponding to the number of conduits to be joined, wherein the conduit ends are located at the respective conduit end-receiving openings, wherein support means (5a, 5b, 5c) is used to support the so located conduit ends whilst being secured to the connecting piece, and wherein the so located conduit ends are secured to the respective conduit end-receiving openings whilst being supported by the support means, characterized in that said support means is inserted into and withdrawn from, respectively, the connecting piece through a sealable opening (6) in the connecting piece, which sealable opening is provided exclusively for inserting and withdrawing, respectively, of the support means therethrough and which sealable opening is then sealed, whereby all of the so located conduit ends can be secured to the respective conduit end-receiving openings whilst being supported by the support means from inside the connecting piece.

2. A method as claimed in claim 1, wherein the conduit ends are secured to the connecting piece (1) by welding.

3. A method as claimed in claim 1, wherein the conduit ends and the connecting piece (1) are formed of thermoplastics.

4. A method as claimed in any of claims 1—3, wherein the conduits (3a, 3b, 3c) are tubes.

5. A method as claimed in any of claims 1—4, characterized in that the sealable opening (6) is a slot.

6. A method as claimed in claim 5, wherein the connecting piece is elongate, characterized in that the slot (6) extends longitudinally of the connecting piece.

7. A method as claimed in any of claims 1—6, characterized in that the sealable opening (6) is sealed using flaps (7a, 7b) extending integrally from the connecting piece.

8. A method as claimed in claim 7, characterized in that the flaps (7a, 7b) are formed of thermoplastics, and the sealable opening (6) is sealed by heating and pressing the flaps together over the opening (6).

9. A method as claimed in claim 2, characterized in that the support means (5a, 5b, 5c) constitutes an electrode which is used in welding the conduit ends to the connecting piece.

10. A method as claimed in any of the preceding claims, wherein the conduit end-receiving openings in the connecting piece for communication with the conduits are defined by open tubular end portions (4a, 4b, 4c) of said connecting piece and the conduit ends are tubular and are inserted into or surround the respective tubular end portions of the connecting piece.

11. A one-piece tubular connecting piece for carrying out the method according to claim 1, which comprises conduit end-receiving openings (4a, 4b, 4c) in a number corresponding to the number of conduits (3a, 3b, 3c) to be joined, characterized in that the connecting piece comprises a sealable opening (6) which is provided exclusively for inserting and withdrawing, respectively, of the support means therethrough and which is then intended to be sealed, whereby all of the so located conduit ends can be secured to the respective conduit end-receiving openings whilst being supported by the support means from inside the connecting piece.

12. A connecting piece as claimed in claim 11, characterized in that the sealable opening (6) is a slot.

13. A connecting piece as claimed in claim 12, wherein the connecting piece (1) is elongate, characterized in that the slot (6) extends longitudinally of the connecting piece.

14. A connecting piece as claimed in claim 11, characterized in that flaps (7a, 7b) are formed integrally with the connecting piece for use in sealing the sealable opening (6).

15. A connecting piece as claimed in claim 13 or 14, characterized in that two flaps (7a, 7b) extend longitudinally at respective sides of the slot (6) for use in sealing the slot.

16. A connecting piece as claimed in claim 15, characterized in that the opposed surfaces (8a, 8b) of the flaps (7a, 7b) are plane convex surfaces, whereby the said surfaces smoothly mutually diverge outwardly from the slot (6).

17. A connecting piece as claimed in claim 11, characterized in that the sealable opening (6) is located opposite a conduit end-receiving opening (4b) in the connecting piece for a conduit end.

18. A connecting piece as claimed in claim 11, wherein the conduit end-receiving openings (4a, 4b, 4c) in the connecting piece for the conduit ends are tubular and portions of the connecting piece (1) adapted to surround or be surrounded by the respective conduit ends (3a, 3b, 3c).

19. A connecting piece as claimed in claim 18, wherein the central axis of said tubular end portions (4a, 4b, 4c) are substantially co-planar and the connecting piece (1) has a Y- or T-shaped axial section in said plane.

20. A connecting piece as claimed in any of claims 11—19, formed of thermoplastics.

**Revendications**

1. Procédé de connexion de deux ou plusieurs tubes (3a, 3b, 3c) au moyen d'une pièce tubulaire de connexion d'un seul tenant (1) présentant des ouvertures de réception d'extrémités de tubes (4a, 4b, 4c en nombre correspondant au nombre de tubes à raccorder, ledit procédé consistant à disposer les extrémités des tubes dans les ouvertures respectives de réception des extrémités de tubes, des moyens de support (5a, 5b, 5c) étant utilisés pour supporter les extrémités de tubes ainsi disposées lors de leur fixation à la pièce de connexion, et à fixer les extrémités de tubes ainsi disposées sur les ouvertures respectives de réception des extrémités de tubes tandis qu'elles sont supportées par les moyens de support, ledit procédé étant caractérisé en ce que lesdits moyens de support sont introduits dans la pièce de connexion et en sont retirés par une ouverture obturable séparée (6) de la pièce de connexion, conçue exclusivement pour l'insertion et le retrait respectif des moyens de support, après quoi ladite ouverture séparée soudable est soudée, de sorte que toutes les extrémités de tubes ainsi disposées peuvent être fixées aux ouvertures respectives tout en étant suportées par les moyens de support de l'intérieur de la pièce de connexion.

2. Procédé suivant la revendication 1, caractérisé en ce que les extrémités des tubes sont fixées sur la pièce de connexion (1) par soudage.

3. Procédé suivant la revendication 1, caractérisé en ce que les extrémités des tubes et la pièce de connexion (1) sont en matière thermoplastique.

4. Procédé suivant l'une quelconque des revendications 1 à 3, caractérisé en ce que les tubes (3a, 3b, 3c) constituent des tubulures.

5. Procédé suivant l'une quelconque des revendications 1 à 4, caractérisé en ce que l'ouverture obturable, notamment par soudure (6) est une fente.

6. Procédé suivant la revendication 5, selon lequel la pièce de connexion est de forme allongée, caractérisé en ce que la fente (6) s'étend longitudinalement dans la pièce de connexion.

7. Procédé suivant l'une quelconque des revendications 1 à 6, caractérisé en ce que l'ouverture soudable (6) est obturée au moyen de volets (7a, 7b) formés d'un seul tenant avec la pièce de connexion.

8. Procédé suivant la revendication 7, caractérisé en ce que les volets (7a, 7b) sont en matière thermoplastique et en ce que l'ouverture (6) est obturée par chauffage et pression des volets l'un contre l'autre par dessus l'ouverture (6).

9. Procédé suivant la revendication 2, caractérisé en ce que les moyens de support (5a, 5b, 5c) constituent une électrode qui est utilisée pour le soudage des extrémités des tubes sur la pièce de connexion.

10. Procédé suivant l'une des revendications 1 à 9, caractérisé en ce que les ouvertures de réception d'extrémité de tube de la pièce de connexion devant communiquer avec lesdits tubes sont constituées par des portions tubulaires d'extrémité (4a, 4b, 4c) de ladite pièce de connexion, et en ce que les extrémités des tubes sont tubulaires et sont enfilées dans ou sur les portions tubulaires d'extrémité respectives de la pièce de connexion.

11. Pièce tubulaire de connexion d'un seul tenant pour la mise en oeuvre du procédé de la revendication 1, ladite pièce présentant des ouvertures de réception d'extrémité de tubes (4a, 4b, 4c) en nombre correspondant au nombre de tubes (3a, 3b, 3c) à raccorder, ladite pièce de connexion étant caractérisée en ce qu'elle présente une ouverture séparée soudable (6), conçue exclusivement pour l'insertion et le retrait, respectivement, des moyens de support par ladite ouverture séparée, après quoi elle est destinée à être soudée, de sorte que toutes les extrémités de tubes ainsi disposées peuvent être fixées aux ouvertures de réception d'extrémité de tubes tout en étant supportées par les moyens de support de l'intérieur de la pièce de connexion.

12. Pièce de connexion suivant la revendication 11, caractérisée en ce que l'ouverture (6) est constituée par une fente.

13. Pièce de connexion suivant la revendication 12, ladite pièce (1) étant de forme allongée, caractérisée en ce que la fente (6) s'étend longitudinalement dans ladite pièce.

14. Pièce de connexion suivant la revendication 11, caractérisée en ce que des volets (7a, 7b) sont formés d'un seul tenant avec la pièce de connexion en vue de l'obturation par soudage de l'ouverture soudable (6).

15. Pièce de connexion suivant les revendications 13 ou 14, caractérisée en ce que les deux volets (7a, 7b) s'étendent longitudinalement de chaque côté de la fente (6) en vue du soudage de ladite fente.

16. Pièce de connexion suivant la revendication 15, caractérisée en ce que les faces opposées (8a, 8b) des volets (7a, 7b) présentent des surfaces places-convexes, telles que lesdites surfaces divergent doucement l'une de l'autre vers l'extérieur à partir de la fente (6).

17. Pièce de connexion suivant la revendication 11, caractérisée en ce que l'ouverture soudable (6) est située à l'opposé d'une ouverture de réception d'extrémité de tube (4b) de la pièce de connexion.

18. Pièce de connexion suivant la revendication 11, caractérisée en ce que les ouvertures de réception d'extrémité de tube (4a, 4b, 4c), de la pièce de connexion sont tubulaires, et en ce que des portions de la pièce de connexion (1) sont agencées de manière à entourer, ou à être entourées par les extrémités respectives des tubes (3a, 3b, 3c).

19. Pièce de connexion suivant la revendication 18, caractérisée en ce que les axes centraux des portions tubulaires d'extrémité (4a, 4b, 4c) sont situés sensiblement dans un même plan et en ce que la pièce de connexion (1) présente une section axiale en forme de Y ou de T dans ledit plan.

20. Pièce de connexion suivant l'une quelconque des revendications 11 à 19, caractérisée en ce qu'elle est en matière thermoplastique.

**Patentansprüche**

1. Verfahren zum Verbinden zweier oder mehrerer Rohre (3a, 3b, 3c) mittels eines einstückigen, rohrförmigen Anschlußstückes (1) mit Rohrendaufnahmeöffnungen (4a, 4b, 4c) mit einer Anzahl, welche der Anzahl der zu verbindenden Rohre entspricht, wobei die Rohrenden an den entsprechenden Rohrendaufnahmeöffnungen angeordnet sind, eine Stützeinrichtung (5a, 5b, 5c) verwendet wird, um die so angeordneten Rohrenden zu haltern, während sie an dem Verbindungsstück befestigt werden, und wobei die so angeordneten Rohrenden an den entsprechenden Rohrendaufnahmeöffnungen befestigt werden, während sie von der Stützeinrichtung gehalten werden, dadurch gekennzeichnet, daß die Stützeinrichtung in das Anschlußstück durch eine abdichtbare Öffnung (6) im Anschlußstück in dieses eingeführt bzw. aus diesem herausgezogen wird, daß die abdichtbare Öffnung ausschließlich zum Einführen bzw. Herausziehen der Stützeinrichtung durch das Loch hindurch vorgesehen ist und daß die abdichtbare Öffnung dann abdichtend verschlossen wird, wodurch alle so angeordneten Rohrenden an den entsprechenden Rohrendaufnahmeöffnungen befestigt werden, während sie von innerhalb des Anschlußstückes von der Stützeinrichtung gehaltert werden.

2. Verfahren nach Anspruch 1, dadurch gekennzeichnet, daß die Rohrenden durch Schweißen an dem Anschlußstück (1) befestigt werden.

3. Verfahren nach Anspruch 1, dadurch gekennzeichnet, daß die Rohrenden und das Anschlußstück (1) aus Thermoplast gebildet sind.

4. Verfahren nach einem der Ansprüche 1 bis 3, dadurch gekennzeichnet, daß die Leitungen (3a, 3b, 3c) Rohre oder Schläuche sind.

5. Verfahren nach einem der Ansprüche 1—4, dadurch gekennzeichnet, daß die abdichtbare Öffnung (6) ein Schlitz ist.

6. Verfahren nach Anspruch 5, bei welchem das Anschlußstück länglich ist, dadurch gekennzeichnet, daß sich der Schlitz (6) längs zum Anschlußstück erstreckt.

7. Verfahren nach einem der Ansprüche 1—6, dadurch gekennzeichnet, daß die abdichtbare Öffnung (6) dadurch dichtend geschlossen wird, daß Laschen (7a, 7b) verwendet werden, die einstückig aus dem Anschlußstück ragen.

8. Verfahren nach Anspruch 7, dadurch gekennzeichnet, daß die Laschen (7a, 7b) aus Thermoplast gebildet sind und die abdichtbare Öffnung (6) durch Erwärmen und Zusammendrücken der Laschen über die Öffnung (6) dichtend verschlossen wird.

9. Verfahren nach Anspruch 2, dadurch gekennzeichnet, daß die Stützeinrichtung (5a, 5b, 5c) eine Elektrode bildet, die beim Anschweißen der Leitung- bzw. Rohrenden an das Anschlußstück verwendet wird.

10. Verfahren nach einem der vorhergehenden Ansprüche, dadurch gekennzeichnet, daß die Rohrendaufnahmeöffnungen im Anschlußstück für die Verbindung mit den Rohren durch offene, rohrförmige Endteile (4a, 4b, 4c) des Anschlußstückes gebildet werden und die Rohrenden rohrförmig sind und in die entsprechenden rohrförmigen Endteile des Anschlußstückes eingeführt werden oder diese umgreifen.

11. Einstückiges, rohrförmiges Anschlußstück zur Durchführung des Verfahrens nach Anspruch 1, welches Rohrendaufnahmeöffnungen (4a, 4b, 4c) in einer Anzahl aufweist, welche der Anzahl der zu verbindenden Rohre (3a, 3b, 3c) entspricht, dadurch gekennzeichnet, daß das Anschlußstück eine abdichtbare Öffnung (6) aufweist, die ausschließlich zum Einführen bzw. Herausziehen der Stützeinrichtung durch das Loch vorgesehen ist und dann dafür bestimmt ist, abgedichtet zu werden, wodurch alle der so angeordneten Rohrenden an den entsprechenden Rohrendaufnahmeöffnungen befestigt werden können, während sie von der Stützeinrichtung von innerhalb des Anschlußstückes gehaltert werden.

12. Anschlußstück nach Anspruch 11, dadurch gekennzeichnet, daß die abdichtbare Öffnung (6) ein Schlitz ist.

13. Anschlußstück nach Anspruch 12, wobei das Anschlußstück (1) länglich ist, dadurch gekennzeichnet, daß sich der Schlitz (6) längs zum Anschlußstück erstreckt.

14. Anschlußstück nach Anspruch 11, dadurch gekennzeichnet, daß Laschen (7a, 7b) einstückig mit dem Anschlußstück für die Verwendung beim Abdichten der abdichtbaren Öffnung (6) gebildet sind.

15. Anschlußstück nach Anspruch 13 oder 14, dadurch gekennzeichnet, daß sich zwei Laschen (7a, 7b) längs an entsprechenden Seiten des Schlitzes (6) für die Verwendung beim Abdichten des Schlitzes erstrecken.

16. Anschlußstück nach Anspruch 15, dadurch gekennzeichnet, daß die gegenüberliegenden Oberflächen (8a, 8b) der Laschen (7a, 7b) plan-konvexe Oberflächen sind, wodurch die Oberflächen vom Schlitz (6) nach außen gegenseitig gleichmäßig auseinanderlaufen.

17. Anschlußstück nach Anspruch 11, dadurch gekennzeichnet, daß die abdichtbare Öffnung (6) gegenüber einer Rohrendaufnahmeöffnung (4b) im Anschlußstück für ein Rohrende angeordnet ist.

18. Anschlußstück nach Anspruch 11, dadurch gekennzeichnet, daß die Rohrendaufnahmeöffnungen (4a, 4b, 4c) im Anschlußstück für die Rohrenden rohrförmig sind und Teile des Anschlußstückes (1) sind, die geeignet derart ausgestaltet sind, daß sie die entsprechenden Rohrenden (3a, 3b, 3c) umgreifen oder von diesen umgriffen werden.

19. Anschlußstück nach Anspruch 18, dadurch gekennzeichnet, daß die Mittenachse der rohrförmigen Endteile (4a, 4b, 4c) im wesentlichen koplanar, d.h. in einer Ebene liegend sind und daß das Anschlußstück (1) einen Y- oder T-förmigen Axialschnitt in dieser Ebene hat.

20. Anschlußstück nach einem der Ansprüche 11—19, welches aus Thermoplast geformt ist.

# Fig. 1

# Fig. 2

0 015 897

# Fig. 3

# Fig. 4